# FASCICULE DE BREVET EUROPEEN

(11) **EP 2 242 373 B1**
(45) Date de publication et mention de la délivrance du brevet: **18.02.2015**
(21) Numéro de dépôt: 08872207.9
(22) Date de dépôt: 03.12.2008
(51) Int. Cl.: A23C 9/123

(54) **UTILISATION DE L. CASEI SSP. PARACASEI COMME ANTIFONGIQUE**
VERWENDUNG VON L. CASEI SSP. PARACASEI ALS ANTIFUNGALES MITTEL
USE OF L. CASEI SSP. PARACASEI AS ANTIFUNGAL AGENT

(30) Priorité: 04.12.2007 FR 0708451
(43) Date de publication de la demande: 27.10.2010
(73) Titulaire: Compagnie Gervais Danone, 75009 Paris (FR)
(72) Inventeur: PERRIER, Louise, F-91270 Vigneux sur Seine (FR); LOYSANCE-PAROUX, Catherine, F-35520 La Meziere (FR); TIRILLY, Yves, F-29470 Plougastel-Daoulas (FR); FUHRMANN, Benoit, F-78690 Les Essarts Le Roi (FR)
(74) Mandataire: Marcadé, Véronique
(86) Numéro de dépôt international: PCT/FR2008/001679
(87) Numéro de publication internationale: WO 2009/098411

(56) Documents cités:
- EP-A- 0 302 300
- EP-A- 1 308 506
- EP-A- 1 683 425
- WO-A-97/36603
- FR-A- 2 775 420
- GOURAMA, H.: "Inhibition of Growth and Mycotoxin Production of Penicillium by Lactobacillus Species" LEBENSMITTEL-WISSENSCHAFT UND -TECHNOLOGIE, vol. 30, no. 3, 1997, pages 279-283, XP002488076
- MIESCHER ET AL.: "A Mixed Culture of Propionibacterium jensenii and Lactobacillus paracasei subsp. paracasei Inhibits Food Spoilage Yeasts" SYSTEM. APPL. MICROBIOL., vol. 27, no. 2, 1 janvier 2004 (2004-01-01), pages 229-237, XP002542508
- DATABASE FSTA INTERNATIONAL FOOD INFORMATION SERVICE (IFIS), FRANkFURT-MAIN, DE; 2007, Tuma et al.: "Isolation of antifungally active lactobacilli from Edam cheese" XP002542509 Database accession no. 2008-00-p1429

## Description

La présente invention est relative à l'utilisation de la souche CNCM I-1518 de *Lactobacillus casei* pour protéger un produit laitier contre le développement des moisissures.

L'utilisation de microorganismes sélectionnés dans le but d'améliorer la conservation des aliments est connue depuis des millénaires. Parmi ces microorganismes, on citera les bactéries lactiques, qui sont largement utilisées pour la conservation de produits laitiers, de produits carnés, ou de produits d'origine végétale, destinés à l'alimentation humaine ou animale. L'amélioration de la conservation des aliments par les bactéries lactiques repose en grande partie sur leur capacité à produire, au cours de la fermentation, de l'acide lactique et d'autres acides organiques, qui inhibent la croissance des microorganismes indésirables (bactéries et moisissures) en abaissant le pH du milieu, le rendant défavorable à la croissance de ces microorganismes, et/ou qui ont un effet toxique direct sur ceux-ci. Outre ces acides organiques, certaines bactéries lactiques produisent des substances anti-fongiques et/ou anti-bactériennes, telles que le peroxyde d'hydrogène, le diacétyle, ou la nisine.

La Demande EP 0221499 décrit des propriétés antifongiques de *Lactobacillus casei* ssp. *rhamnosus* NRRL-B-15972. Lorsque cette bactérie est cultivée sur milieu d'agar additionné de jus de concombre, elle est capable d'inhiber totalement la croissance de *Chaetomium olivacium,* et partiellement celle *d'Aspergillus terreus* et de *Verticillium* sp. En revanche, dans ces conditions, elle n'a aucun effet sur la croissance de *Penicillium oxalicum ;* un effet inhibiteur sur ce microorganisme est toutefois observé avec des cultures de cette bactérie sur jus de concombre, à un pH de l'ordre de 3,7.

La Demande EP 0576780 décrit les effets antifongiques de *Lactobacillus casei* ssp. *rhamnosus* LC-705. Des cultures de cette bactérie, obtenues sur un milieu à base de lactosérum, additionné d'hydrolysat de caséine et d'extrait de levure, peuvent inhiber la croissance de *Penicillium, Cladosporium, Fusarium,* et *Candida.*

La Demande PCT WO 97/36603 décrit les effets antifongiques de *Lactobacillus casei* ssp. *casei* N94/49432. Des cultures de cette bactérie, obtenues sur un milieu synthétique additionné d'extrait de levure, ont un effet antifongique sur *Alternaria, Chaetomtum, Cladosporium, Colletotrichum, Cunninghamella, Dothiorella, Geotrichum, Phoma,* et *Phomopsis.*

La Demande EP 1308506 décrit les effets antifongiques et antibactériens d'une combinaison de *Propionibacterium jensenii* avec certaines souches de *Lactobacillus casei* ssp. *paracasei.*

Les Inventeurs ont maintenant constaté que des bactéries de la souche CNCM I-1518 de l'espèce *Lactobacillus casei* ssp. *paracasei* possédaient une activité antifongique, notamment sur les moisissures du genre *Penicillium,* lorsqu'elles étaient utilisées isolément, c'est-à-dire sans être associées à d'autres bactéries antifongiques, et notamment avec *Propionibacterium jensenii.*

Cette activité antifongique présente les caractéristiques suivantes :
Elle ne se développe pas lorsque la souche CNCM I-1518 *L. casei* est cultivée sur milieu synthétique, mais apparaît lorsqu'elle est cultivée sur du lait, ou un milieu à base de lait, à partir de 5 heures de culture. Elle devient maximale après 24 à 48h de fermentation.

Elle est sensible au pH. Elle disparait en dessous de pH 4, mais ré-apparaît toutefois si le pH est remonté au dessus de 4.

Elle est également sensible à la chaleur. Elle disparait après un traitement thermique de 1 minute à 60°C.

Elle n'est d'autre part pas liée à la production d'acides organiques tels que les acides sorbique, propionique ou benzoïque (qui sont connus pour leur activité antifongique), ni à celle de peroxyde d'hydrogène, ces substances n'étant pas détectables dans les cultures de la souche CNCM I-1518 dotées de propriétés anti-fongiques.

La présente invention a pour objet l'utilisation d'une bactérie de la souche CNCM I-1518 de l'espèce *L*. *casei* ssp. *paracasei* pour conférer des propriétés antifongiques à un produit laitier fermenté.

En particulier, la présente invention a pour objet un procédé pour inhiber le développement d'une moisissure de la classe des Ascomycètes, et notamment du genre *Penicillium,* dans un produit laitier fermenté, caractérisé en ce qu'il comprend la fermentation d'un substrat laitier en présence d'une bactérie de la souche CNCM I-1518 pendant un temps suffisant pour l'apparition dans le substrat d'une activité antifongique. Cette activité antifongique a pour effet d'inhiber le développement de ladite moisissure en cas de contamination dudit produit par celle-ci postérieurement à la fermentation.

Avantageusement, la quantité de ladite bactérie de la souche CNCM I-1518 utilisée pour ensemencer le substrat laitier pour ladite fermentation est d'au moins 10⁶, de préférence au moins 10⁷, et avantageusement comprise entre 10⁷ et 10⁸ u.f.c. par gramme de substrat laitier.

Selon un mode de mise en oeuvre préféré du procédé conforme à l'invention, la fermentation dudit substrat laitier est effectuée pendant au moins 5 heures, de préférence pendant au moins 15 heures, et de manière tout à fait préférée pendant 24 à 48 heures. Pour obtenir une activité antifongique en utilisant des temps de fermentation courts, il est préférable d'utiliser une quantité relativement importante de la souche CNCM I-1518 ; par exemple, pour observer une activité antifongique après 5 heures de fermentation du substrat laitier, l'ensemencement sera effectué à raison d'au moins 10⁷ u.f.c. par gramme de substrat laitier.

Selon un autre mode de mise en oeuvre préféré du procédé conforme à l'invention, il comprend, si nécessaire l'ajustement du pH du substrat fermenté à une valeur supérieure à 4, de préférence comprise entre 4 et 6,5.

La souche CNCM I-1518 est décrite notamment dans la Demande EP0794707 ; elle a été déposée, selon les dispositions du Traité de Budapest, le 30 décembre 1994, auprès de la CNCM (Collection Nationale de Cultures de Microorganismes), 25 rue du Docteur Roux, à Paris.

La présente invention sera mieux comprise à l'aide du complément de description qui va suivre, qui se réfère à des exemples illustrant l'activité antifongique de la souche CNCM I-1518.

### EXEMPLE 1 : MISE EN ÉVIDENCE DE L'ACTIVITÉ ANTIFONGIQUE DE PRODUITS LAITIERS FERMENTÉS EN PRÉSENCE DE L. CASEI SSP. PARACASEI.

Un produit laitier fermenté contenant *L. casei* ssp. *paracasei* (Actimel®, contenant la souche CNCM I-1518 associée à *S. thermophilus* et *L. bulgaricus),* et un produit laitier fermenté contenant *S. thermophilus* et *L. bulgaricus* (Témoin) ont été inoculés avec 6 espèces fongiques à raison de 10² spores: *Mucor circinelloides* (2 souches testées) ; *Mucor plumbeus ; Pénicillium expansum ; Pénicillium roqueforti ; Penicillium brevicompactum.*

Ces espèces sont représentatives des contaminations habituellement rencontrées sur les produits laitiers frais.

Les produits sont ensuite incubés à 10°C et examinés tous les 7 jours pour l'apparition de thalles de croissance fongique.

On observe un développement important des espèces de *Mucor* sur les deux produits, dès 14 jours dans le cas d'Actimel®, et dès 7 jours dans le cas du produit témoin. En revanche, on observe une croissance extrêmement faible (agrégats) ou une absence de croissance visible de *Penicillium* sur Actimel®, après 40 jours d'incubation, alors que l'on observe un envahissement de la surface du produit Témoin en seulement 14 jours.

Dans une seconde série d'expérimentations, l'activité antifongique de la souche CNCM I-1518 a été évaluée par une technique dérivée de celle des antibiogrammes. Elle consiste à géloser le produit à tester (80% de produit + 20% de solution d'agar à 15g/l) et à couler le mélange en boite de Pétri.

Sur le milieu solidifié, on dépose 3 disques en papier stériles sur lesquels sont déposées 100 spores de la moisissure à tester (*P. expansum.* LMSA 00 083)

Les boites de Pétri sont incubées à 25°C et à 10°C. Le diamètre du thalle fongique est mesuré régulièrement.

Les produits qui ont été testés sont les suivants : Actimel® ; mix laitier d'Actimel® avec *S. thermophilus* et *L. bulgaricus,* sans la souche CNCM I-1518 ; mix laitier d'Actimel® sans *S. thermophilus* et *L. bulgaricus,* fermenté pendant 24 heures en présence de la souche CNCM I-1518.

Les résultats de deux essais pour chacune des conditions testées sont illustrés dans le Tableau I ci-dessous.

**TABLEAU I**

| | Diamètre de thalle après incubation (mm) | | Diamètre de thalle (mm) après 17 jours à 10° | | Diamètre de thalle après 24 jours à 10° | |
|---|---|---|---|---|---|---|
| | Essai 1 8jours à 25°C | Essai 2 7jours à 25°C | Essai 1 | Essai 2 | Essai 1 | Essai 2 |
| Actimel® Standard (commerce) | 17,6 ± 4,4 | 19,2 ± 4,8 | 9,6 ± 0,5 | 10 ± 0,9 | 10,9 ± 0,8 | 12,9 ± 1,8 |
| Actimel® sans *L.casei* | >65 | >65 | >65 | >65 | >65 | >65 |
| Mix laitier d'Actimel® fermenté 24h avec *L*. *casei* en l'absence de *S*. *thermophilus* et *L. bulgaricus* | 9,4 ± 1 | 8,8 ± 1 | 11,4 ± 1 | 15,1 ± 1,2 | 14,1 ± 0,9 | 17,2 ± 1,2 |

Les deux produits contenant la souche CNCM I-1518 (Actimel® ; et le mix laitier d'Actimel® fermenté 24h avec *L*. *casei* en l'absence de *S. thermophilus* et *L. bulgaricus* présentent une activité antifongique, contrairement au produit qui ne la contient pas (mix laitier d'Actimel® sans *L*. *casei*).

### EXEMPLE 2 : CINETIQUE D'APPARITION DE L'ACTIVITE ANTIFONGIQUE AU COURS DE LA FERMENTATION PAR L. CASEI SSP. PARACASEI.

Des tests d'activité antifongique ont été effectués sur un mélange (Mix) constitué de lait écrémé, additionné de 2% de glucose et de 6% de protéines laitières (Protéines de lait écrémé classiques), ensemencé avec 10⁷cfu/ml de la souche CNCM I-1518 sous forme lyophilisée. Des échantillons de mélange ont été prélevés avant et après inoculation par CNCM I-1518, puis après 15h, 24h, 48h, et 72h de fermentation. Préalablement aux tests d'activité antifongique, les échantillons prélevés et congelés ont été irradiés à 10 kG afin de bloquer le développement des bactéries ceci afin d'éviter leur développement et métabolisme au cours du test. Les échantillons à tester sont préalablement décongelés et le pH des échantillons a été ajusté à 6,2 (pH du mélange de départ). Les tests ont été effectués comme décrit à l'Exemple 1 ci-dessus.

Les résultats de ces tests (mesure du diamètre du thalle) sont illustrés par le Tableau II ci-dessous. Les mentions « Thalle sur disque » et « Agrégats », se réfèrent à des traces de croissance fongique, observées uniquement sur le disque.

**TABLEAU II**

| **Test 7 jours à 25°C** | | | | | |
|---|---|---|---|---|---|
| Mix non ensemencé | Mix + *L. casei* | | | | |
| | To | T15h | T24h | T48h | T72h |
| 36,8mm ± 10,2 | 22,5mm ± 3,1 | 19,3 mm ± 2,7 | Agrégats | Agrégats | Agrégats |

| **Test 19 jours à 10°C** | | | | | |
|---|---|---|---|---|---|
| Mix non ensemencé | Mix + *L*. *casei* | | | | |
| | To | T15h | T24h | T48h | T72h |
| 48,5mm ± 1,9 | 13,8mm ± 3,3 | Thalle sur disque | Agrégats | Agrégats | Agrégats |

Ces essais montrent l'apparition précoce de l'activité antifongique, qui semble parfois faiblement présente dès l'inoculation mais n'apparaît nettement qu'à partir de 15h de fermentation et devient maximale entre 24h et 48h. Cette activité est toujours présente après 48h et même 72h sous réserve que le pH soit supérieur à 4.

### EXEMPLE 3 : SENSIBILITE DE L'ACTIVITE ANTIFONGIQUE AU pH

Des tests d'activité antifongique ont été effectués dans les mêmes conditions que dans l'Exemple 2 ci-dessus, mais sans ajustement du pH préalablement aux tests.

Les résultats sont illustrés par le Tableau III ci-dessous.

**TABLEAU III**

| | Mix non ensemencé | Mix + *L. casei* | | | | |
|---|---|---|---|---|---|---|
| | | To | T15h | T24h | T48h | T72h |
| pH | 6,32 | 6,01 | 5,63 | 4,8 | 3,94 | 3,75 |
| | 33,2 ± 2,5 | 12.8 ± 1.3 | Agrégats | Agrégats | 34,3 ± 11,4 | 25,2 ± 2,1 |

Ces résultats montrent que le pouvoir antifongique est altéré lorsque le pH est inférieur à 4.

Cette altération semble toutefois réversible, le pouvoir antifongique étant restauré si le pH est ramené à une valeur supérieure à 4.

### EXEMPLE 4 : INFLUENCE DU MILIEU DE FERMENTATION SUR L'ACTIVITE ANTI-FONGIQUE.

Afin de déterminer si l'activité antifongique était liée au milieu de culture, des essais ont été effectués en utilisant comme milieu de culture d'une part le mélange laitier décrit à l'exemple 2, et d'autre part un milieu synthétique, de composition suivante :peptone 10gr/L ; extrait de viande 10gr/L ; extrait de levure 5gr/L ; Phosphate bipotassique 2gr/L ; Citrate d'ammonium 2gr/L ; Sulfate de magnésium 0,1gr/L ; Sulfate de manganèse 0,05 gr/L ; Glucose 20gr/L ; Tween 80 :1ml ; qsp 1 L eau permutée. Ces milieux ont été ensemencés avec 10⁷ cfu/ml de la souche CNCM I-1518 sous forme lyophilisée. Après 24 et 48 heures de culture, l'activité antifongique a été testée, comme décrit à l'Exemple 2, pendant 7 jours à 25°C et 20 jours à 10°C.

Lorsque la culture est effectuée sur le mélange laitier, on observe une inhibition complète de la croissance fongique quelque soit la température du test. En revanche, aucune inhibition n'est observée lorsque la culture est effectuée sur le milieu synthétique, ceci pour une population bactérienne équivalente.

## Revendications

1. Utilisation d'une bactérie de l'espèce *L. casei* ssp. *paracasei* pour conférer des propriétés antifongiques à un produit laitier fermenté, ladite bactérie de l'espèce *L. casei* ssp. *paracasei* étant une bactérie de la souche CNCM I-1518.

2. Procédé pour inhiber le développement d'une moisissure de la classe des Ascomycètes dans un produit laitier fermenté, **caractérisé en ce qu'**il comprend la fermentation d'un substrat laitier en présence d'une bactérie de l'espèce *L. casei* ssp. *paracasei* pendant un temps suffisant pour l'apparition dans le substrat d'une activité antifongique, ladite bactérie de l'espèce *L*. *casei* ssp. *paracasei* étant une bactérie de la souche CNCM I-1518.

3. Procédé selon la revendication 2, **caractérisé en ce que** la quantité de ladite bactérie de l'espèce *L. casei* ssp. *paracasei* utilisée pour ensemencer le substrat laitier pour ladite fermentation est d'au moins 10⁶ u.f.c. par gramme de substrat laitier.

4. Procédé selon la revendication 3, **caractérisé en ce que** la quantité de ladite bactérie de l'espèce *L. casei* ssp. *paracasei* utilisée pour ensemencer le substrat laitier pour ladite fermentation est d'au moins 10⁷ u.f.c. par gramme de substrat laitier.

5. Procédé selon une quelconque des revendications 2 à 4, **caractérisé en ce que** la fermentation dudit substrat laitier est effectuée pendant au moins 5 heures.

6. Procédé selon une quelconque des revendications 2 à 5, **caractérisé en ce qu'**il comprend, si nécessaire, l'ajustement du pH du substrat fermenté à une valeur supérieure à 4.

7. Procédé selon une quelconque des revendications 2 à 6, **caractérisé en ce que** ladite moisissure de la classe des Ascomycètes appartient au genre *Penicillium.*

## Patentansprüche

1. Verwendung eines Bakteriums der Gattung *L. casei* ssp. *paracasei,* um einem fermentierten Milchprodukt antifungale Eigenschaften zu verleihen, wobei das Bakterium der Gattung *L. casei* ssp. *paracasei* ein Bakterium des Stamms CNCM 1-1518 ist.

2. Verfahren zum Inhibieren der Entwicklung eines Schimmelpilzes der Klasse der Ascomyceten in einem fermentierten Milchprodukt, **dadurch gekennzeichnet, dass** es die Fermentation eines Milchsubstrates in Gegenwart eines Bakteriums der Gattung *L. casei* ssp. *paracasei* während einer Zeit, die ausreichend für das Auftreten einer antifungalen Aktivität in dem Substrat ist, umfasst, wobei das Bakterium der Gattung *L. casei* ssp. *paracasei* ein Bakterium des Stamms CNCM 1-1518 ist.

3. Verfahren gemäß Anspruch 2, **dadurch gekennzeichnet, dass** die Menge des Bakteriums der Gattung *L. casei* ssp. *paracasei,* die verwendet wird, um das Milchsubstrat für die genannte Fermentation zu beimpfen, wenigstens 10⁶ KbE pro Gramm Milchsubstrat ist.

4. Verfahren gemäß Anspruch 3, **dadurch gekennzeichnet, dass** die Menge des Bakteriums der Gattung *L. casei* ssp. *paracasei,* die verwendet wird, um das Milchsubstrat für die genannte Fermentation zu beimpfen, wenigstens 10⁷ KbE pro Gramm Milchsubstrat ist.

5. Verfahren gemäß einem der Ansprüche 2 bis 4, **dadurch gekennzeichnet, dass** die Fermentation des Milchsubstrates während wenigstens 5 Stunden durchgeführt wird.

6. Verfahren gemäß einem der Ansprüche 2 bis 5, **dadurch gekennzeichnet, dass** es, wenn notwendig, die Einstellung des pH des fermentierten Substrates auf einen Wert von über 4 umfasst.

7. Verfahren gemäß einem der Ansprüche 2 bis 6, **dadurch gekennzeichnet, dass** der Schimmelpilz der Klasse der Ascomyceten zu der Art *Penicillium* gehört.

## Claims

1. Use of a bacterium of the species *L. casei ssp. paracasei* to impart antifungal properties to a fermented dairy product, said bacterium of the species *L. casei ssp. paracasei* being a bacterium of the CNCM 1-1518 strain.

2. Method for inhibiting the development of a mould of the class Ascomycetes in a fermented dairy product, **characterised in that** it includes the fermentation of a dairy substrate in the presence of a bacterium of the species *L. casei ssp. paracasei* for a sufficient time for antifungal activity to appear in the substrate, said bacterium of the species *L. casei ssp. paracasei* being a bacterium of the CNCM I- 1518 strain.

3. Method according to claim 2, **characterised in that** the quantity of said bacterium of the species *L. casei ssp. paracasei* used to inoculate the dairy substrate for said fermentation is at least 10⁶ CFU per gram of dairy substrate.

4. Method according to claim 3, **characterised in that** the quantity of said bacterium of the species *L. casei ssp. paracasei* used to inoculate the dairy substrate for said fermentation is at least 10⁷ CFU per gram of dairy substrate.

5. Method according to any one of claims 2 to 4, **characterised in that** the fermentation of said dairy substrate is carried out for at least 5 hours.

6. Method according to any one of claims 2 to 5, **characterised in that** it includes, if necessary, adjusting the pH of the fermented substrate to a value greater than 4.

7. Method according to any one of claims 2 to 6, **characterised in that** said mould of the class Ascomycetes belongs to the genus *Penicillium.*
